# EUROPÄISCHE PATENTANMELDUNG

(11) **EP 1 443 447 A2**
(43) Veröffentlichungstag der Anmeldung: **04.08.2004**
(21) Anmeldenummer: 03025241.5
(22) Anmeldetag: 05.11.2003
(51) Int. Cl.: G06F 19/00

(54) **Drahtloses System und Verfahren zum Einfügen von kryptographischen Daten**

(30) Priorität: 22.01.2003 DE 10302586
(71) Anmelder: CompuGroup Holding AG, 56070 Koblenz (DE)
(72) Erfinder: Broer, Jan, 56072 Koblenz (DE)
(74) Vertreter: Richardt, Markus Albert

(57) **Zusammenfassung**

Die Erfindung betrifft ein Verfahren zur Erzeugung einer Datenbank zur Speicherung von Rezeptdaten mit folgenden Schritten:
- Erfassung und Speicherung von Rezeptdaten in einem ersten Rechenzentrum,
- Auswahl eines zweiten Rechenzentrums aus einer Menge von Rechenzentren, wobei jedes Rechenzentrum der Menge von Rechenzentren einer Krankenkasse zugeordnet ist, wobei die Auswahl anhand von die Krankenkasse identifizierenden Rezeptdaten erfolgt,
- Übertragung der Rezeptdaten an das zweite Rechenzentrum und Speicherung der Rezeptdaten in dem zweiten Rechenzentrum,
- Übertragung von in den Rechenzentren der Menge von Rechenzentren gespeicherten Rezeptdaten an ein drittes Rechenzentrum,
- Erzeugung der Datenbank in dem dritten Rechenzentrum durch Speicherung der von der Menge von Rechenzentren empfangenen Rezeptdaten.

## Beschreibung

Die vorliegende Erfindung betrifft ein Verfahren zur Erzeugung einer Datenbank zur Speicherung von Rezeptdaten für die Zwecke der Durchführung einer Interaktionsprüfung von Medikamenten, ein digitales Speichermedium mit einem entsprechenden Computerprogramm, ein Computersystem zur Erzeugung einer solchen Datenbank sowie ein Praxis-EDV-System zur Durchführung von Interaktionsprüfungen.

Aus dem Stand der Technik sind Arzneimittel- und Informationsdatenbanken für niedergelassene Ärzte bekannt. Solche Datenbanken erlauben es auch Interaktionen zwischen Medikamenten zu prüfen. Die Datenbank ifap Index® (http://www.ifap-index.de/arztdb/index.html) der ifap Service-Institut für Ärzte und Apotheker GmbH beinhaltet eine Funktion für eine solche Interaktionsprüfung, die auch als Interaktionen-Check bezeichnet wird. Mittels dieser Interaktionsprüfung werden die von dem behandelnden Arzt für die Rezeptierung ausgewählten Arzneimittel vor dem Rezeptdruck auf Interaktionen hin überprüft. Diese Software ermöglicht auch die Berücksichtigung von Angaben des Patienten zu anderen Medikamenten, die er oder sie einnimmt. Hierzu gibt es ein entsprechendes Eingabefenster.

Nachteilig an einer solchen vorbekannten EDV-Funktion zur Durchführung einer Interaktionsprüfung ist, dass die Prüfung nur die von dem behandelnden Arzt selbst für die Rezeptierung ausgewählten Medikamente und die Angaben des Patienten umfassen kann. Der betreffende Patient kann aber noch bei weiteren Ärzten in Behandlung sein, die auch Ihrerseits Arzneimittel verordnet haben. Entsprechend sind die Angaben des Patienten u.U. unvollständig oder fehlerhaft. Solche von anderen Ärzten verordneten Medikamente können bei der vorbekannten Interaktionsprüfung nicht mit der erforderlichen Sicherheit berücksichtigt werden, da es an einer hierfür erforderlichen globalen Datenbasis fehlt.

Der Erfindung liegt daher die Aufgabe zu Grunde ein verbessertes Verfahren zur Erzeugung einer Datenbank zur Speicherung von Rezeptdaten für die Durchführung von Interaktionsprüfungen zu schaffen, sowie ein verbessertes Verfahren zur Durchführung von Interaktionsprüfungen auf der Grundlage einer solchen Datenbank, ein digitales Speichermedium mit einem entsprechenden Computerprogramm, ein Computersystem zur Erzeugung einer solchen Datenbank und ein Praxis-EDV-System mit einer verbesserten Interaktionsprüfungsfunktion.

Die der Erfindung zu Grunde liegenden Aufgaben werden jeweils mit den Merkmalen der unabhängigen Patentansprüche gelöst. Bevorzugte Ausführungsformen der Erfindung sind in den abhängigen Patenansprüchen angegeben.

Die vorliegende Erfindung ermöglicht die Erzeugung einer globalen Datenbank, in der die von verschiedenen Ärzten einem Patienten verordneten Medikamente gespeichert sind. Im Vergleich zum Stand der Technik hat eine solche Datenbank den Vorteil, dass sie vollständig ist und ein hohes Maß an Aktualität und Datenintegrität gewährleistet, so dass Interaktionsprüfungen vollständig durchführbar sind, und nicht nur die von einem bestimmten Arzt ausgewählten Medikamente umfassen. Eine solche globale Interaktionsprüfung bei der Verordnung von Medikamenten durch einen behandelnden Arzt hat den Vorteil, dass sich die Risiken und Nebenwirkungen bei der Medikamentierung eines Patienten reduzieren lassen.

Erfindungsgemäß erfolgt die Erzeugung einer solchen Datenbank für globale Interaktionsprüfungen so, dass zunächst das Rezept in einer Apotheke eingelöst wird, das Rezept von der Apotheke an ein Apotheken-Rechenzentrum weitergeleitet wird, und dort eine Erfassung der Rezeptdaten erfolgt. Zu den Rezeptdaten gehören beispielsweise eine Medikamentenkennung, insbesondere die sogenannte Pharmazentralnummer (PZN), eine Patientenkennung, insbesondere die Versichertennummer des Patienten, und eine Krankenkassenkennung zur Identifizierung der Krankenkasse, bei der der Patient versichert ist, insbesondere die Versicherungsnummer der Krankenkasse. Vorzugsweise wird ferner das Rezeptdatum erfasst. Der betreffende Datensatz wird in dem Apotheken-Rrechenzentrum gespeichert.

In dem Apotheken-Rrechenzentrum ist eine Liste der Krankenkassen-Rechenzentren gespeichert. Jede Krankenkasse hat zumindest ein Rechenzentrum, welches unter anderem für die Zwecke der Abrechnung dient. Die Adressen der Krankenkassen-Rechenzentren sind ebenfalls in dem Apotheken-Rechenzentrum gespeichert. Bei diesen Adressen kann es sich zum Beispiel um Internet-Adressen für die Datenübertragung über das Internet handeln. Beispielsweise kann auf die Adresse eines Krankenkassen-Rechenzentrums mit der Krankenkassenkennung, insbesondere der Versicherungsnummer, als Schlüssel zugegriffen werden.

Von dem Apotheken-Rechenzentrum werden die Datensätze der Rezeptdaten an die betreffenden Krankenkassen-Rechenzentren übertragen. Die Krankenkassen-Rechenzentren können solche Datensätze von verschiedenen Apotheken-Rechenzentren erhalten. Die Erzeugung einer globalen Datenbasis, die sämtliche relevante Rezeptdaten von Patienten beinhaltet, erfolgt nun so, dass die Datensätze von sämtlichen Krankenkassen-Rechenzentren an ein einziges zentrales Rechenzentrum übertragen werden, in dem die Datensätze abgespeichert werden. Dadurch resultiert eine Datenbank, in der für jeden Patienten sämtliche Rezeptdaten der verschiedenen behandelnden Ärzte gespeichert sind, wobei auf diese Rezeptdaten mit der Patientenkennung, insbesondere der Versichertennummer, als Schlüssel zugegriffen werden kann. Mit Hilfe dieser globalen Datenbasis können Interaktionsprüfungen vollständig durchgeführt werden, indem auch zuvor durch andere Ärzte erfolgte Verordnungen bei der Interaktionsprüfung anlässlich der Rezeptierung von dem Praxis-EDV-System des behandelnden Arztes berücksichtigt werden können.

Nach einer bevorzugten Ausführungsform der Erfindung wird die globale Datenbank auf einen Datenträger gespeichert. Der Datenträger kann dann an die Arztpraxen verteilt werden, so dass in den jeweiligen Praxis-EDV-Systemen jeweils eine lokale Kopie der globalen Datenbank vorliegt. Mit Hilfe von turnusmäßigen Updates werden die lokalen Kopien der globalen Datenbank aktualisiert.

Zusätzlich zu der globalen Datenbank kann auf dem Datenträger auch ein Update der Praxis-EDV-Software gespeichert sein. Gleichzeitig mit den turnusmäßigen Updates der Praxis-EDV-Software kann also gleichzeitig die lokale Kopie der globalen Datenbank auf den neuesten Stand gebracht werden. Dies verringert den Aufwand für die Verteilung der Datenträger. Als Datenträger kommen insbesondere Datenträger mit hoher Speicherdichte, insbesondere optische Speicher, vorzugsweise CD oder DVD in Frage.

Nach einer bevorzugten Ausführungsform der Erfindung wird nicht die komplette globale Datenbank auf dem Datenträger gespeichert, sondern es werden für die verschiedenen Arztpraxen Teil-Datenbanken aus der globalen Datenbank erzeugt. Im Allgemeinen hat eine Arztpraxis ein bestimmtes Einzugsgebiet von zum Beispiel nicht mehr als 100 km. Die Erzeugung einer Teil-Datenbank aus der globalen Datenbank erfolgt mit Hilfe einer Filterfunktion, die die Datensätze anhand eines Entfernungs- oder Wegzeitkriteriums zwischen dem Patientenwohnsitz und der Arztpraxis filtert.

Die für eine bestimmte Arztpraxis erzeugte Teil-Datenbank der globalen Datenbank beinhaltet also nur diejenigen Datensätze von Patienten, die innerhalb eines vorgegebenen Umkreises, das heißt einer vorgegebenen maximalen Entfernung oder maximalen Wegzeit, von der Arztpraxis ihren Wohnsitz haben. Eine solche Filterfunktion ist beispielsweise realisierbar, indem die Adresse des Patienten mit in dem Rezeptdatensatz erfasst wird und ferner die Adressen der Arztpraxen in dem zentralen Rechenzentrum gespeichert sind. Mit Hilfe üblicher Routen Planungsprogramme kann dann die Entfernung oder die Wegzeit zwischen dem Wohnsitz eines Patienten und einer Arztpraxis festgestellt werden. Nur wenn die Entfernung oder die Wegzeit eine vorgegebene maximale Grenze nicht überschreitet, wird der betreffende Datensatz für die Teil-Datenbank der betreffenden Arztpraxis ausgewählt. Auf diese Art und Weise lässt sich das Datenvolumen der globalen Datenbank reduzieren, um es auf einem einzigen Datenträger abspeichern zu können.

Alternativ wird das relevante Territorium, z.B. die Bundesrepublik Deutschland, in eine Anzahl von z.B. 10 Clustern, d.h. geographischen Gebieten, eingeteilt. Für jedes dieser geographischen Gebiete wird dann ein gesonderter Datenträger hergestellt, der an die in dem betreffenden Gebiet ansässigen Arztpraxen verteilt wird. Der Datenträger für ein bestimmtes geographische Gebiet beinhaltet dann eine Teil-Datenbank die diejenigen Patienten abdeckt, die in dem Gebiet ihren Wohnsitz haben. Dies hat den Vorteil, daß sich die Herstellung der Datenträger erheblich verbilligt, insbesondere beim Einsatz von optischen Datenträgern.

Nach einer weiteren bevorzugten Ausführungsform der Erfindung werden keine lokalen Kopien der globalen Datenbank in die Praxis-EDV eingespeist, sondern die Praxis-EDV-Systeme können online auf die zentrale, globale Datenbank zugreifen. Vorzugsweise sind für einen effizienten und schnellen Zugriff Standleitungen zwischen den Praxis-EDV-Systemen und dem zentralen Rechenzentrum vorhanden, insbesondere SDSL-Verbindungen.

Wenn eine Online-Zugriffsmöglichkeit vorhanden ist, werden vorzugsweise auch eine Medikamentendatenbank und eine Interaktionsdatenbank online angeboten, so dass auch insoweit die Speicherung lokaler Kopien in der Praxis-EDV und die Durchführung von Updates entfallen kann. Ein weiterer Vorteil ist, dass damit die Aktualität und die Integrität der Daten weiter erhöht wird.

Zur Durchführung einer Interaktionsprüfung wird also erfindungsgemäß von dem behandelnden Arzt wie folgt vorgegangen:

Zunächst wählt der behandelnde Arzt ein oder mehrere Medikamente zur Verordnung für einen Patienten aus. Diese Auswahl kann aus einer lokalen oder aus einer Online-Medikamenten-Datenbank erfolgen. Zur Durchführung einer Interaktionsprüfung wird dann auf die lokale Kopie der globalen Datenbank oder auf die online angebotene globale Datenbank zugegriffen.

Eine Abfrage der globalen Datenbank erfolgt mit der Patientenkennung, insbesondere der Versichertennummer, als Schlüssel, womit auf sämtliche Rezeptdaten, die für diesen Patienten in der globalen Datenbank gespeichert sind, zugegriffen werden kann. Die Wirkstoffe von bereits zuvor verordneten Medikamenten werden dann mit den Wirkstoffen des für eine Verordnung durch den behandelnden Arzt ausgewählten Medikaments abgeglichen, um mögliche Interaktionsrisiken zu identifizieren. Falls kein solches Risiko ermittelbar ist, erfolgt der Rezeptausdruck; im gegenteiligen Fall erfolgt die Anzeige eines Warnhinweises, so dass der behandelnde Arzt unter Umständen ein alternatives Medikament auswählen kann.

Im folgenden werden bevorzugte Ausführungsformen der Erfindung mit Bezugnahme auf die Zeichnungen näher erläutert. Es zeigen:
- Figur 1: ein Blockdiagramm eines Praxis-EDV-Systems,
- Figur 2: ein Flussdiagramm zur Durchführung einer Interaktionsprüfung,
- Figur 3: ein Blockdiagramm eines Computersystems zur Erzeugung einer globalen Datenbank,
- Figur 4: eine alternative Ausführungsform des Computersystems der Figur 3.

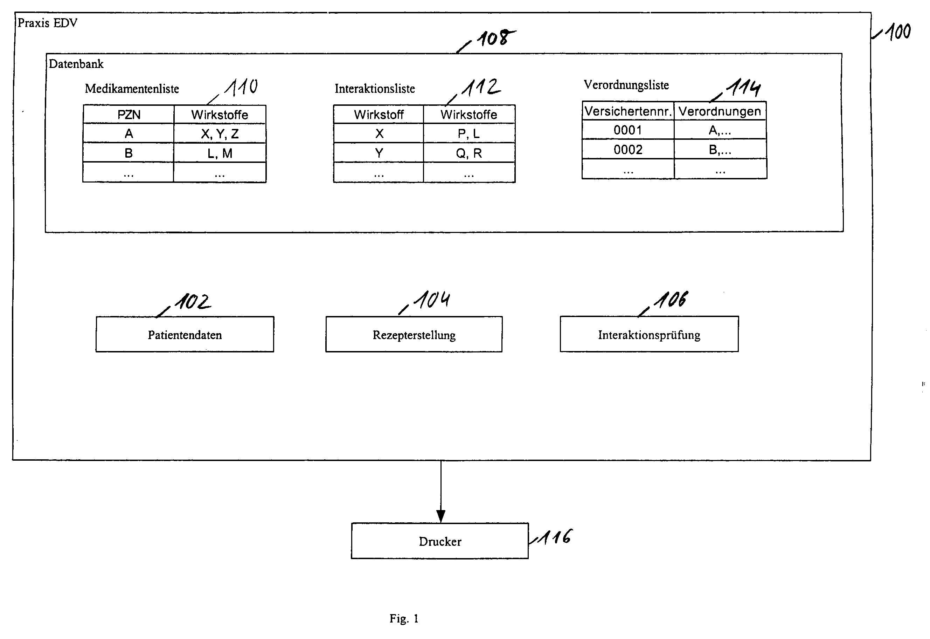

Die Figur 1 zeigt ein Praxis-EDV-System einer Arztpraxis. Das Praxis-EDV-System 100 hat eine Datenbank 102 zur Speicherung von Patientendaten, insbesondere zur Speicherung der sogenannten elektronischen Patientenkartei.

Das Praxis-EDV-System 100 hat ferner ein Modul 104 zur Rezepterstellung und ein Modul 106 für die Durchführung von Interaktionsprüfungen. Ferner hat das Praxis-EDV-System 100 eine Datenbank 108 mit einer Medikamentenliste 110, einer Interaktionsliste 112 und einer Verordnungsliste 114.

Die Medikamentenliste 110 beinhaltet die für eine Verordnung zur Verfügung stehenden Medikamente mit deren jeweiliger Pharmazentralnummer (PZN). Zu jeder PZN sind die in dem betreffenden Medikament beinhalteten Wirkstoffe angegeben. Beispielsweise hat das Medikament mit der PZN "A" die Wirkstoffe X, Y und Z. Auf der Grundlage der Medikamentenliste 110 wird zum Beispiel ein alphabetisch sortiertes Auswahlfenster, insbesondere ein Pulldown oder Popup Menü erzeugt, aus dem der behandelnde Arzt ein oder mehrere Medikamente durch Anklicken auswählen kann. /

In der Interaktionsliste 112 sind diejenigen Wirkstoffe abgespeichert, die in Kombination mit einem vorgegebenen Wirkstoff ein Risiko aufweisen. Beispielsweise soll der Wirkstoff X nicht mit dem Wirkstoff P oder L kombiniert werden, da diese Kombination ein medizinisches Risiko aufweist. Entsprechendes gilt für den Wirkstoff Y, der nicht mit dem Wirkstoff Q oder mit dem Wirkstoff R kombiniert werden soll.

Die Verordnungsliste 114 ist die globale Datenbank oder eine Teil-Datenbank der globalen Datenbank mit den Rezeptdaten der Patienten. Beispielsweise hat der Patient mit der Versichertennummer "0001" bereits eine Verordnung für die Einnahme des Medikaments A und gegebenenfalls weiter dort aufgelisteter Medikamente erhalten. Von Vorteil ist dabei, dass die Verordnungsliste 114 die zuvor erfolgten Verordnungen sämtlicher den betreffenden Patienten behandelnder Ärzte beinhaltet.

Zur Rezepterstellung ruft der behandelnde Arzt das Modul 104 auf, welches auf die Datenbank 102 zum Zugriff auf die Patientenstammdaten und auf die Medikamentenliste 110 zur Auswahl eines oder mehrerer Medikamente durch den behandelnden Arzt zugreift. Nach der Auswahl eines oder mehrerer Medikamente wird das Modul 106 zur Durchführung einer Interaktionsprüfung mit Hilfe der Interaktionsliste 112 und der Verordnungsliste 114 aufgerufen.

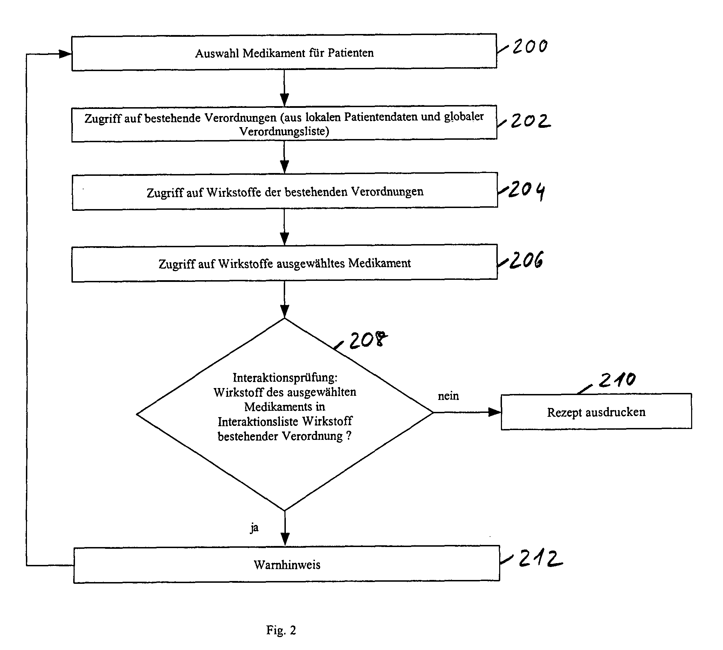

Die Durchführung der Interaktionsprüfung wird im Weiteren mit Bezugnahme auf die Figur 2 näher erläutert. In dem Schritt 200 erfolgt die Auswahl eines Medikaments für einen Patienten durch den behandelnden Arzt mit Hilfe der Medikamentenliste 110. In dem Schritt 202 wird durch das Modul 106 die Interaktionsprüfung durch einen Zugriff auf die Verordnungsliste 114 (vgl. Figur 1) gestartet. Zusätzlich zu einem Zugriff auf die Verordnungsliste 114 kann das Modul 106 auch auf die Datenbank 102 zugreifen. Auf diese Art und Weise können auch Verordnungen erfasst werden, die seit dem Zeitpunkt des letzten Updates der Verordnungsliste 114 durch den behandelnden Arzt vorgenommen worden sind und in der Datenbank 102 abgespeichert worden sind.

In dem Schritt 204 wird auf die Medikamentenliste 110 zugegriffen, um die Wirkstoffe der bestehenden Verordnungen zu ermitteln. Ebenfalls auf die Medikamentenliste 110 wird in dem Schritt 206 zugegriffen, um die Wirkstoffe des zur Verordnung durch den behandelnden Arzt ausgewählten Medikaments zu ermitteln.

In dem Schritt 208 wird für die in den Schritten 204 und 206 ermittelten Wirkstoffe eine Interaktionsprüfung durchgeführt, das heißt es wird geprüft, ob es zu einem Wirkstoff des ausgewählten Medikaments zumindest einen Wirkstoff der bestehenden Verordnungen gibt, der ein Interaktionsrisiko hat. Falls dies der Fall ist, erfolgt in dem Schritt 212 die Ausgabe eines Warnhinweises, und der behandelnde Arzt kann in dem Schritt 200 ein alternatives Medikament auswählen. Falls sich kein Interaktionsrisiko ergibt, erfolgt der Rezeptausdruck auf dem Drucker 116 des Praxis-EDV-Systems 100 (vgl. Fig. 1) in dem Schritt 210.

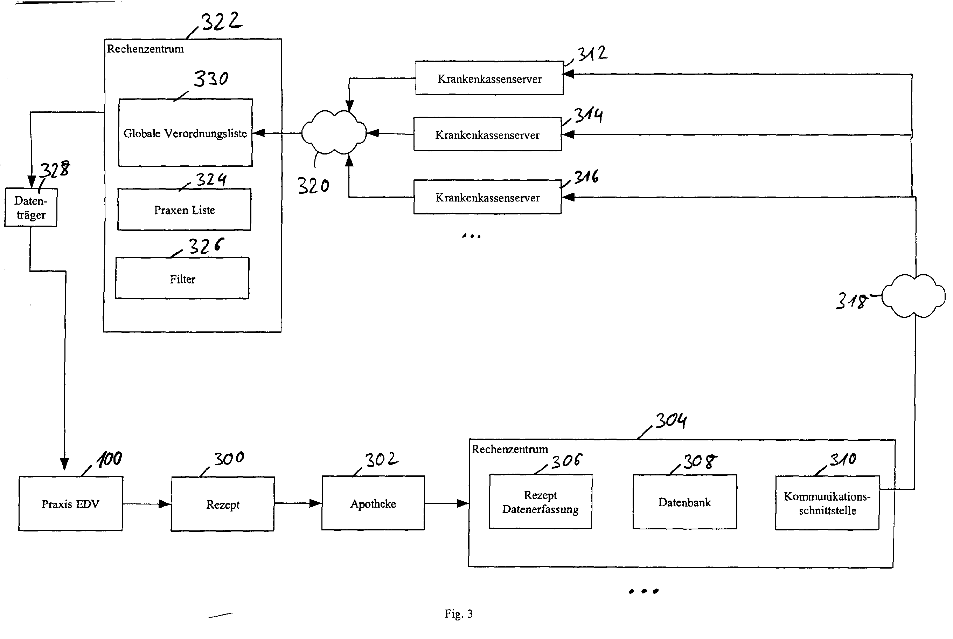

Die Figur 3 zeigt ein Computersystem zur Erzeugung einer globalen Verordnungsliste 330 (vgl. Figur 1). Ein mit Hilfe des Praxis-EDV-Systems 100 erzeugtes Rezept 300 wird von einem Patienten bei einer Apotheke 302 eingelöst.

Dabei kann es sich um eine beliebige Apotheke handeln, das heißt der Patient kann die Apotheke frei auswählen. Der Patient erhält das Medikament von der Apotheke und die Apotheke gibt das Rezept an ein Apotheken-Rechenzentrum 304 weiter. Das Rechenzentrum 304 hat ein Modul 306 zur Rezeptdatenerfassung, beispielsweise einen Scanner mit OCR-Zeichenerkennung, eine Datenbank 308 zur Speicherung der erfassten Rezeptdaten sowie eine Kommunikationsschnittstelle 310. Zur flächendeckenden Versorgung von Apotheken mit solchen Apotheken-Rechenzentren 304 können mehrere solche Rechenzentren verteilt vorhanden sein. Die Apotheke 302 erhält üblicherweise die Zahlung für das Rezept 300 von dem Rechenzentrum 304 und das Rechenzentrum 304 nimmt die Abrechnung gegenüber der jeweiligen Krankenkasse vor.

Jede Krankenkasse hat zumindest einen Krankenkassenserver 312, 314, 316... Beispielsweise gehört der Krankenkassenserver 312 der AOK, der Krankenkassenserver 314 der Techniker Krankenkasse, der Krankenkassenserver 316 der Barmer Ersatzkasse usw.

Wenn der Patient beispielsweise bei der Techniker Krankenkasse versichert ist, ist eine entsprechende Angabe in das Rezept 300 eingedruckt. Die Angabe der Krankenkasse, bei der der Patient versichert ist, wird in dem Modul 306 mit erfasst. Beispielsweise ist die Angabe der Krankenversicherung in dem Rezept 300 in Klartext eingedruckt. Nach Einscannen und OCR in dem Modul 306 wird der Angabe der Krankenkasse die Versicherungsnummer dieser Krankenkasse zugeordnet und zusammen mit dem erfassten Datensatz gespeichert.

Ferner wird durch einen weiteren Datenbankzugriff die Adresse des Krankenkassenservers 314 der Techniker Krankenkasse ermittelt, so dass der Datensatz von der Kommunikationsschnittstelle 310 des Rechenzentrums 304 an den Krankenkassenserver 314 für die Zwecke der Abrechnung gegenüber dem Rechenzentrum 304 übertragen werden kann. Vorzugsweise erfolgt die Übertragung über ein Netzwerk 318, vorzugsweise das Internet.

Aufgrund der Abrechnung des Rechenzentrums 304 gegenüber den verschiedenen Krankenkassen und der Abrechnung weiterer Apotheken-Rechenzentren des Typs vom Rechenzentrum 304, die in der Figur 3 nicht gezeigt sind, liegen in den Krankenkassenservern 312, 314, 316, ... verteilt, sämtliche Rezeptdaten der versicherten Patienten vor. Die entsprechenden Datensätze werden von den Krankenkassenservern 312, 314, 316, ... über ein Netzwerk 320, beispielsweise das Internet an ein Rechenzentrum 322 übertragen. Dort werden die verschiedenen Datensätze der Krankenkassenserver 312, 314, 316, ... zu der globalen Verordnungsliste 330) konsolidiert. Die globale Verordnungsliste 330 beinhaltet also für jeden Patienten eine vollständige Liste der Medikamente, die von verschiedenen Ärzten verordnet worden sind.

Das Rechenzentrum 322 hat ferner ein Modul 324, in dem eine Liste der Arztpraxen gespeichert ist, die von dem Rechenzentrum 322 bedient werden. Jeder der Arztpraxen ist dabei in dem Modul 324 eine Adresse zugeordnet oder andere geocodierte Daten, die zur Bestimmung der geographischen Position der Arztpraxis dienen. Mittels eines Filters 326 können für jede der Arztpraxen, die in der Liste des Moduls 324 gespeichert sind, diejenigen Patienten aus der globalen Verordnungsliste 330 ausgefiltert werden, die sich innerhalb eines vorgegebenen Umkreises im Bereich der betreffenden Arztpraxis befinden, das heißt zum Beispiel im Umkreis von 100 km oder nicht mehr als eine Stunde Fahrtzeit entfernt.

Die ausgefilterten Patienten mit den zugehörigen Verordnungsdaten werden dann als Teil-Liste der globalen Verordnungsliste 114 auf einem Datenträger 328 gespeichert, der dann zum Beispiel per Post an die betreffende Arztpraxis verschickt wird. Dort wird der Datenträger 328 in das Praxis-EDV-System 100 eingespeist, um die lokale Kopie der Verordnungsliste (vgl. Verordnungsliste 114) zu aktualisieren. Die Aktualisierung der Verordnungsliste kann dabei mit einer Aktualisierung der Praxis-EDV-Software einhergehen.

Die Erzeugung einer Teil-Liste aus der globalen Verordnungsliste 330 hat dabei den Vorteil, dass sich das Datenvolumen reduzieren lässt, so dass sich die Teil-Liste besser auf einem einzigen Datenträger 328 abspeichern lässt. Die beteiligen Arztpraxen erhalten also jeweils einen auf die betreffende Arztpraxis zugeschnittenen Teil der globalen Verordnungsliste 330, der nur diejenigen Patientendaten beinhaltet, die Patienten betreffen, die ihren Wohnsitz in der Nähe der Arztpraxis haben.

Alternativ zu der Erstellung von praxisspezifischen Datenträgern 328 werden in dem Rechenzentrum 322 Teil-Datenbanken für vordefinierte geographische Gebiete erzeugt. Eine solche Teil-Datenbank beinhaltet nur die Rezeptdaten von Patienten, die in dem betreffenden geographischen Gebiet ihren Wohnsitz haben. Der Datenträger mit einer solchen Teil-Datenbank wird an die Arztpraxen in dem betreffenden Gebiet verteilt.

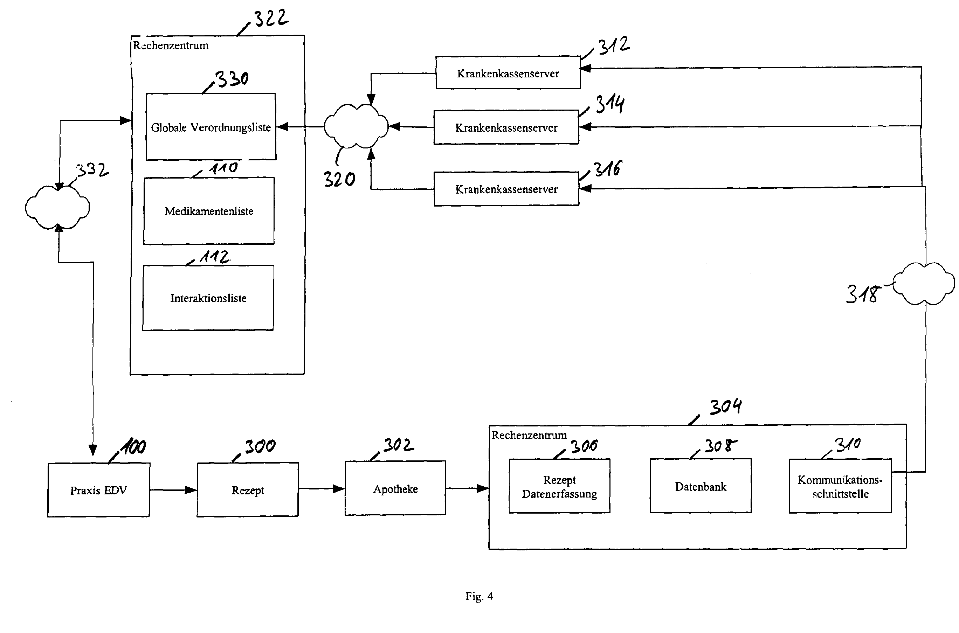

Die Figur 4 zeigt eine alternative Ausführungsform des Systems der Figur 3, wobei einander entsprechende Elemente mit denselben Bezugszeichen gekennzeichnet sind.

Im Unterschied zu der Ausführungsform der Figur 3 ist das Praxis-EDV-System 100 online über das Netzwerk 332, beispielsweise das Internet, vorzugsweise über eine Standleitung, mit dem Rechenzentrum 322 verbunden. Zur Realisierung einer Standleitung wird vorzugsweise eine SDSL-Verbindung aufgebaut.

Im Unterschied zu der Ausführungsform der Figur 1 sind die Medikamentenliste 110 und die Interaktionsliste 112 nicht in dem Praxis-EDV-System 100 gespeichert, sondern sind online über das Netzwerk 332 auf dem Rechenzentrum 322 verfügbar. Dies bedeutet, dass der gesamte Inhalt der Datenbank 108 statt in dem Praxis-EDV-System 100 in dem Rechenzentrum 322 gespeichert ist und ständig online über das Netzwerk 332 für einen Zugriff zur Verfügung steht. Dies hat den Vorteil, dass Updates durch Versendung von Datenträgern entfallen können und dass die Daten besonders aktuell sind. Ferner reduziert diese Vorgehensweise den Speicherbedarf und die erforderlichen Hardware-Ressourcen des Praxis-EDV-Systems 100. Auch Software Updates für das Praxis-EDV-Systems 100 können auf dem Rechenzentrum 322 zum automatischen Download zur Verfügung gestellt werden.

### Bezugszeichenliste

- 100: Praxis-EDV-System
- 102: Datenbank
- 104: Modul
- 106: Modul
- 108: Datenbank
- 110: Medikamentenliste
- 112: Interaktionsliste
- 114: Verordnungsliste
- 116: Drucker
- 300: Rezept
- 302: Apotheke
- 304: Rechenzentrum
- 306: Modul
- 308: Datenbank
- 310: Kommunikationsschnittstelle
- 312: Krankenkassenserver
- 314: Krankenkassenserver
- 316: Krankenkassenserver
- 318: Netzwerk
- 320: Netzwerk
- 322: Rechenzentrum
- 324: Modul
- 326: Filter
- 328: Datenträger
- 330: Verordnungsliste
- 332: Netzwerk

## Patentansprüche

1. Computersystem zur Erzeugung einer Datenbank (114; 330) zur Speicherung von Rezeptdaten mit:
- Mitteln (306) zur Erfassung und Speicherung von Rezeptdaten in einem ersten Rechenzentrum (304),
- Mitteln (308) zur Auswahl eines zweiten Rechenzentrums aus einer Menge von Rechenzentren (312, 314, 316, ...), wobei jedes Rechenzentrum der Menge von Rechenzentren einer Krankenkasse zugeordnet ist, wobei die Auswahl anhand von die Krankenkasse identifizierenden Rezeptdaten erfolgt,
- Mitteln (310) zur Übertragung der Rezeptdaten an das zweite Rechenzentrum und Speicherung der Rezeptdaten in dem zweiten Rechenzentrum,
- Mitteln (320) zur Übertragung von in den Rechenzentren der Menge von Rechenzentren gespeicherten Rezeptdaten an ein drittes Rechenzentrum (322),
- Mitteln zur Erzeugung der Datenbank (330) in dem dritten Rechenzentrum durch Speicherung der von der Menge von Rechenzentren empfangenen Rezeptdaten.

2. Computersystem nach Anspruch 1, wobei die Rezeptdaten eine Medikamentenkennung beinhalten, und wobei es sich bei der Medikamentenkennung vorzugsweise um die Pharmazentralnummer des Medikaments handelt.

3. Computersystem nach Anspruch 1 oder 2, wobei in dem ersten Rechenzentrum die Adressen der zweiten Rechenzentren der Menge von Rechenzentren gespeichert sind, und auf die Adressen mit den die Krankenkasse identifizierenden Rezeptdaten als Schlüssel zugegriffen werden kann.

4. Computersystem nach einem der vorhergehenden Ansprüche 1 bis 3, mit einer Liste (324) von Arztpraxen mit Ortsangabe und mit einer Filterfunktion (326) zur Erzeugung einer Teil-Datenbank, die die Rezeptdaten von Patienten beinhaltet, die sich innerhalb eines vorgegebenen Umkreises um eine vorgegebene Arztpraxis befinden.

5. Computersystem nach einem der vorhergehenden Ansprüche 1 bis 4, wobei das dritte Rechenzentrum eine Interaktionsdatenbank (11) aufweist, und auf das dritte Rechenzentrum von einer Praxis-EDV aus online zugegriffen werden kann, um eine Interaktionsprüfung durchzuführen.

6. Verfahren zur Erzeugung einer Datenbank zur Speicherung von Rezeptdaten mit folgenden Schritten:
- Erfassung und Speicherung von Rezeptdaten in einem ersten Rechenzentrum,
- Auswahl eines zweiten Rechenzentrums aus einer Menge von Rechenzentren, wobei jedes Rechenzentrum der Menge von Rechenzentren einer Krankenkasse zugeordnet ist, wobei die Auswahl anhand von die Krankenkasse identifizierenden Rezeptdaten erfolgt,
- Übertragung der Rezeptdaten an das zweite Rechenzentrum und Speicherung der Rezeptdaten in dem zweiten Rechenzentrum,
- Übertragung von in den Rechenzentren der Menge von Rechenzentren gespeicherten Rezeptdaten an ein drittes Rechenzentrum,
- Erzeugung der Datenbank in dem dritten Rechenzentrum durch Speicherung der von der Menge von Rechenzentren empfangenen Rezeptdaten.

7. Verfahren nach Anspruch 6, wobei die Rezeptdaten eine Medikamentenkennung beinhalten, und wobei es sich bei der Medikamentenkennung vorzugsweise um die Pharmazentralnummer des Medikaments handelt.

8. Verfahren nach Anspruch 6 oder 7, wobei in dem ersten Rechenzentrum die Adressen der zweiten Rechenzentren der Menge von Rechenzentren gespeichert sind, und auf die Adressen mit den die Krankenkasse identifizierenden Rezeptdaten als Schlüssel zugegriffen werden kann.

9. Verfahren nach einem der vorhergehenden Ansprüche 6 bis 8, wobei es sich bei den die Krankenkasse identifizierenden Rezeptdaten um die Versicherungsnummer der Krankenkasse handelt und wobei die Rezeptdaten vorzugsweise das Datum des Rezepts beinhalten.

10. Verfahren nach einem der vorhergehenden Ansprüche 6 bis 9, wobei die Rezeptdaten eine eindeutige Patientenkennung beinhalten und die eindeutige Patientenkennung als Schlüssel für den Zugriff auf die Rezeptdaten des betreffenden Patienten in der Datenbank dient.

11. Verfahren nach Anspruch 10, wobei es sich bei der eindeutigen Patientenkennung um die Versichertennummer handelt.

12. Verfahren nach einem der vorhergehenden Ansprüche 6 bis 11, wobei die Datenbank ganz oder teilweise auf Datenträgern gespeichert wird, die zur Verteilung an Praxis-EDV-Systeme vorgesehen sind.

13. Verfahren nach Anspruch 12, wobei in dem dritten Rechenzentrum eine Liste der Arztpraxen mit Ortsangabe vorhanden ist, und mit Hilfe einer Filterfunktion eine Teil-Datenbank aus der Datenbank für eines der Praxis-EDV-Systeme generiert wird, indem diejenigen Patienten und deren Rezeptdaten aus der Datenbank ausgefiltert werden, die ihren Wohnsitz innerhalb eines vorgegebenen Umkreises um die betreffende Arztpraxis haben.

14. Verfahren nach einem der vorhergehenden Ansprüche 6 bis 13, wobei das dritte Rechenzentrum eine Interaktionsdatenbank aufweist, und auf das dritten Rechenzentrum von einer Praxis-EDV aus online zugegriffen wird, um eine Interaktionsprüfung anhand der Datenbank und der Interaktionsdatenbank durchzuführen.

15. Verfahren zur Interaktionsprüfung mit folgenden Schritten:
- Auswahl eines ersten Medikaments für einen Patienten aus einer Medikamenten-Datenbank,
- Abfrage von Rezeptdaten des Patienten aus einer Datenbank,
- Durchführung der Interaktionsprüfung hinsichtlich des ersten Medikaments und zweiter Medikamente, die durch die Rezeptdaten identifiziert werden, mittels einer Interaktionsdatenbank, wobei die Datenbank folgendermaßen erzeugt worden ist:
- Erfassung und Speicherung von Rezeptdaten eines Rezepts in einem ersten Rechenzentrum,
- Auswahl eines zweiten Rechenzentrums aus einer Menge von Rechenzentren, wobei jedes Rechenzentrum der Menge von Rechenzentren einer Krankenkasse zugeordnet ist, wobei die Auswahl anhand von die Krankenkasse identifizierenden Rezeptdaten erfolgt,
- Übertragung der Rezeptdaten an das zweite Rechenzentrum und Speicherung der Rezeptdaten in dem zweiten Rechenzentrum,
- Übertragung von in den Rechenzentren der Menge von Rechenzentren gespeicherten Rezeptdaten an ein drittes Rechenzentrum,
- Erzeugung der Datenbank in dem dritten Rechenzentrum durch Speicherung der von der Menge von Rechenzentren empfangenen Rezeptdaten.

16. Verfahren nach Anspruch 15, wobei die Datenbank lokal in einer Praxis-EDV gespeichert ist.

17. Verfahren nach Anspruch 15, wobei auf die Datenbank von einer Praxis-EDV aus online zugegriffen wird.

18. Verfahren nach Anspruch 15, 16 oder 17, wobei auf die Interaktionsdatenbank von einer Praxis-EDV online zugegriffen wird.

19. Verfahren nach einem der vorhergehenden Ansprüche 15 bis 18, wobei auf eine Medikamentendatenbank für die Auswahl des ersten Medikaments online zugegriffen wird.

20. Digitales Speichermedium, insbesondere optisches Speichermedium, beispielsweise CD oder DVD, mit einer Datenbank, die wie folgt erzeugt worden ist:
- Erfassung und Speicherung von Rezeptdaten eines Rezepts in einem ersten Rechenzentrum,
- Auswahl eines zweiten Rechenzentrums aus einer Menge von Rechenzentren, wobei jedes Rechenzentrum der Menge von Rechenzentren einer Krankenkasse zugeordnet ist, wobei die Auswahl anhand von die Krankenkasse identifizierenden Rezeptdaten erfolgt,
- Übertragung der Rezeptdaten an das zweite Rechenzentrum und Speicherung der Rezeptdaten in dem zweiten Rechenzentrum,
- Übertragung von in den Rechenzentren der Menge von Rechenzentren gespeicherten Rezeptdaten an ein drittes Rechenzentrum,
- Erzeugung der Datenbank in dem dritten Rechenzentrum durch Speicherung der von der Menge von Rechenzentren empfangenen Rezeptdaten.

21. Praxis-EDV-System mit einer nach einem Verfahren gemäß einem der Patentansprüche 6 bis 14 erzeugten Datenbank.
